# EUROPEAN PATENT APPLICATION

(11) **EP 1 090 961 A1**
(43) Date of publication of application: **11.04.2001**
(21) Application number: 00308768.1
(22) Date of filing: 05.10.2000
(51) Int. Cl.: C09B 23/08, B41M 5/40, G11B 7/24

(54) **Cyanine dyes**

(30) Priority: 06.10.1999 JP 28512399; 07.03.2000 JP 2000062572; 12.09.2000 JP 2000275764
(71) Applicant: KABUSHIKI KAISHA HAYASHIBARA SEIBUTSU KAGAKU KENKYUJO, Okayama-shi Okayama (JP)
(72) Inventor: Hohsaka, Ayako, Okayama-shi, Okayama (JP); Matsuura, Dai, Okayama-shi, Okayama (JP); Kawata, Toshio, Okayama-shi, Okayama (JP); Yasui, Shigeo, Okayama-shi, Okayama (JP)
(74) Representative: Daniels, Jeffrey Nicholas

(57) **Abstract**

Disclosed are novel unsymmetrical indolenin pentamethine cyanine dyes, and light absorbents and optical recording media, which comprise the cyanine dyes. The cyanine dyes exert excellent recording features when used in high-speed-recordable-type optical recording media.

## Description

### Background of the Invention

### Field of the Invention

The present invention relates to novel cyanine dyes, more particularly, to unsymmetrical indolenin pentamethine cyanine dyes that exert excellent recording features when used in high-speed-recordable-type optical recording media.

### Description of the Prior Art

As coming into this multi-media age, the following optical recording media have been greatly focused; a recordable compact disc or CD-R (a write-once memory using compact disc) and a digital video disc or DVD-R (a write-once memory using digital video disc). Optical recording media can be classified roughly into inorganic optical recording media which have recording layers composed of inorganic substances such as tellurium, selenium, rhodium, carbon, or hydrogen sulfide; and organic optical recording media which have recording layers composed of light absorbents which mainly contain organic dye compounds.

Organic optical recording media can be usually prepared by dissolving a cyanine dye in an organic solvent such as 2,2,3,3-tetrafluoro-1-propanol (abbreviated as "TFP" hereinafter); coating the solution on the surface of a polycarbonate substrate; drying the solution to form a recording layer; and closely attaching successively a reflection layer, which is made of an alloy or metal such as gold, silver or copper, onto the surface of the recording layer, and a protection layer, for example, those made of an ultraviolet-ray-hardening-resin, onto the surface of the reflection layer. When compared with inorganic optical recording media, those of organic-ablation-type may have the drawback that environmental lights such as reading- or natural-lights may easily change their recording layer. Organic optical recording media, however, have the merit that they can be made at a lesser cost because the recording layer can be directly formed by coating light absorbents in a solution form on the surface of substrate. Further, organic optical recording media have been mainly used as low-cost media, because;
(i) they are substantially free of corrosion even when contacted with moisture or seawater, since they are mainly composed of organic materials, and
(ii)by using a read-only machine, heat-deformative type optical recording media, one of organic optical recording media can read out informations recorded by a fixed format in the media.

The technologies of high-speed-writing and reading out data in organic optical recording media are urgent key objects for this multi-media age. Recently, a writing-machine, which is writable six times as fast as a normal linear velocity; and a reading-machine, which is readable 15 times as fast as a normal linear velocity, have been put to practical use. A writing-machine, which is writable over six times as fast as the normal linear velocity, has not been easily put to practical use. One of the reasons is that a high-output laser device, which is small in size, low-cost and installable in a normal writing machine, has not yet been put to practical use, although high-speed-writing needs a high-output laser device.

Another reason is a thermal cracking of organic color compounds used in a recording layer. In organic optical recording media, pits are formed by using heat generated when a dye melts and decomposes after absorbing a laser beam or ray (hereinafter abbreviated as "a laser beam"). Many well-known organic color compounds have a melting point and a decomposition point which separate each other. Because of their significantly different temperatures, pits are formed slowly on a recording layer when irradiated by a laser beam and the heat of fusion, and decomposition conducts to spread around the irradiated point and deforms the adjacent pits which have been already formed. This makes it difficult to promptly form desired pits on the limited recording surface of optical recording media.

### Summary of the Invention

In view of the foregoing, the object of the present invention is to provide organic dye compounds, which exert excellent recording features in high-speed-recordable-type optical recording media.

To attain the above object, the present inventors eagerly studied and screened compounds. As a result, they found specific unsymmetrical indolenin pentamethine cyanine dyes,
(i) which have a indolenin- and benzoindolenin-rings at both ends of their pentamethyne chains, and substantially absorb near infrared lights with wavelengths of around 780 nm when formed in a thin layer,
(ii) and which are obtainable through a step of reacting 3,3-dimethyl indolenium compounds having an active methyl group with 2-(1,3-butadienyl)-3,3-dimethyl indolenium compounds having an appropriate leaving group (abbreviated as "cyanine dyes" hereinafter).

They also found that most of the cyanine dyes have only decomposition points or those which are easily undistinguishable from their melting points, have relatively high decomposition points, and promptly decompose at around their decomposition points. The present inventors confirmed that stable pits are quickly formed on the recording surfaces in optical recording media with the cyanine dyes when irradiated by laser beams with wavelengths of around 780 nm. The present invention was made based on the creation of novel organic compounds and the discovery of their industrially useful features.

### Brief Description of the Accompanying Drawings

FIG. 1 is an absorption spectrum of the cyanine dye of the present invention when formed in a thin layer.

FIG. 2 is an absorption spectrum of another cyanine dye of the present invention when formed in a thin layer.

FIG. 3 is an absorption spectrum of a known cyanine dye when formed in a membrane.

FIG. 4 shows the results of DTA and TGA of the cyanine dye of the present invention.

FIG. 5 shows the results of DTA and TGA of the another cyanine dye of the present invention.

FIG. 6 shows the results of DTA and TGA of a known cyanine dye.

### Detailed Description of the Invention

The present invention relates to cyanine dyes represented by Formula 1 or 2.

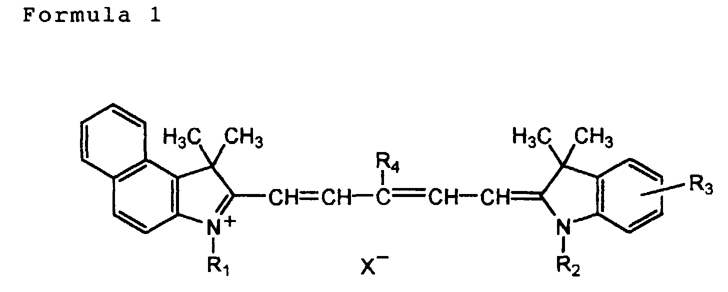

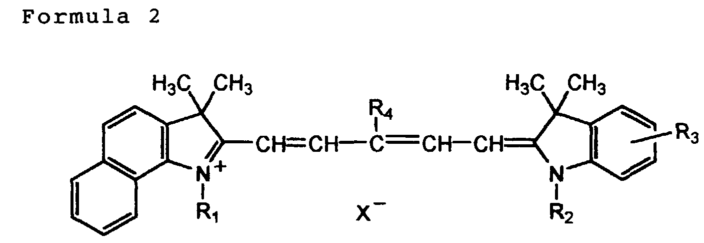

Throughout Formulae 1 and 2, R₁ and R₂ represent an identical or different alkyl group which may have a substituent. Examples of the alkyl group are usually a straight or branched alkyl group having 1 to 5 carbon atoms; lower alkyl groups such as methyl, ethyl, propyl, isopropyl, butyl, isobutyl, sec-butyl, tert-butyl, pentyl, isopentyl, neopentyl, and tert-pentyl groups. Particularly, R₁ preferably represents a straight alkyl group having not more than 4 carbon atoms, and R₂ represents a straight alkyl group having not more than 3 carbon atoms. Substituents of R₁ and R₂ are, for example, halogens such as fluorine and alkoxy groups such as methoxy and ethoxy groups.

R₃ represents a substituent selected from the group consisting of nitro, cyano, trifluoromethyl, trifluoromethoxy, carboxylic acid ester, carboxylic acid amide, alkylaminosulfonyl, and alkylsulfonyl. Carboxylic acid ester in R₃ includes, for example, esters of carbonic acid between lower alcohols or arylalcohols such as methoxycarbonyl, trifluoromethoxycarbonyl, ethoxycarbonyl, propoxycarbonyl, isopropoxycarbonyl, acetoxy, trifluoroacetoxy, phenoxycarbonyl, tolyloxycarbonyl, and benzoyloxy groups. Alkylaminosulfonyl group in R₃ includes, for example, lower alkylaminosulfonyl groups such as metylaminosulfonyl, dimetylaminosulfonyl, ethylaminosulfonyl, diethylaminosulfonyl, propylaminosulfonyl, dipropylaminosulfonyl, isopropylaminosulfonyl, diisopropylaminosulfonyl, butylaminosulfonyl, dibutylaminosulfonyl, and piperidinosulfonyl groups. Alkylsulfonyl group in R₃ includes, for example, lower alkylsulfonyl groups such as metylsulfonyl, ethylsulfonyl, propylsulfonyl, isopropylsulfonyl, and butylsulfonyl groups.

R₄ represents hydrogen or a substituent selected from halogens and methyl group. The halogens may be fluoro, chloro, bromo or iodo, and chloro is particularly desirable.

X⁻ is not particularly limited and can be appropriately selected in view of the solubility of cyanine dyes in organic solvents and the stability of cyanine dyes in recording layers depending on uses. In use of optical recording media, it is usually selected from inorganic acid anions such as phosphoric acid hexafluoride, halogen, phosphoric acid, perchloric acid, periodic acid, antimony hexafluoride, tin acid hexafluoride, fluoroboric acid and tetrafluoroborate ions; organic acid ions such as thiocyanic acid, benzensulfonic acid, naphthalenesulfonic acid, *p*-toluenesulfonic acid, alkylsulfonic acid, benzencarboxylic acid, alkylcarboxylic acid, trihaloalkylcarboxylic acid, alkylsulfonic acid, trihaloalkylsulfonic acid, and nicotinic acid ions; and metal complex ions such as azo, bisphenyldithiol, thiocatechol chelate, thiobisphenolate chelate, and bisdiol-α -diketone. In the cyanine dyes represented by Formulae 1 and 2, any types of their optical isomers of *cis* and *trans* forms may be included in the present invention.

Concrete examples of the present cyanine dyes are those represented by Formulae 1 to 22, which are all unsymmetrical indolenin pentamethine cyanine dyes. These dyes absorb visible lights with wavelengths of around 780 nm when formed in a thin layer. Most of them have only decomposition points or those which are undistinguishable from their melting points, where the decomposition points are not lower than 240 °C. The cyanine dyes have a relatively high thermo-stability and promptly decompose at around their decomposition points. Accordingly, the cyanine dyes exert excellent recording features in optical recording media using laser beams with wavelengths around 780 nm as a wrighting light, particularly high-speed-recordable-type CD-Rs using laser beams with wavelengths of 775-795 nm as a wrighting light.

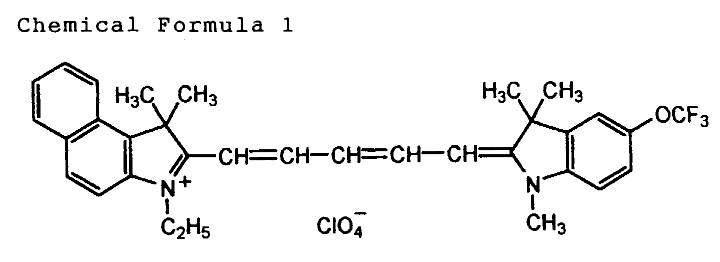

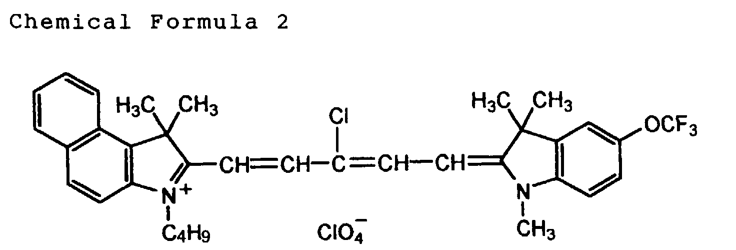

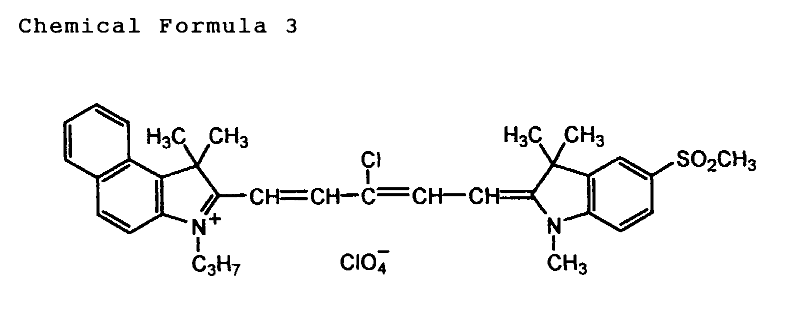

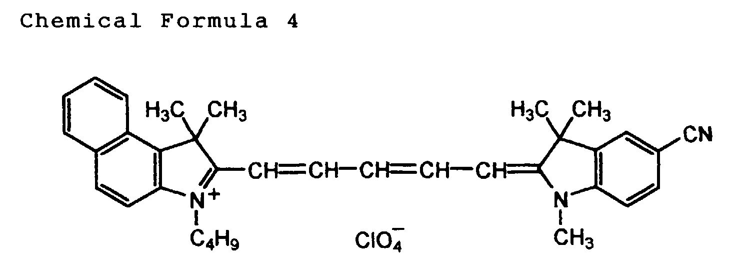

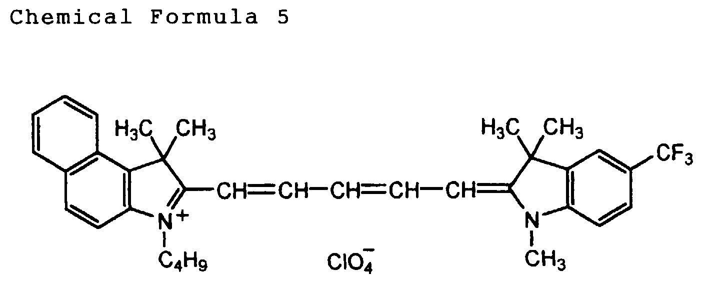

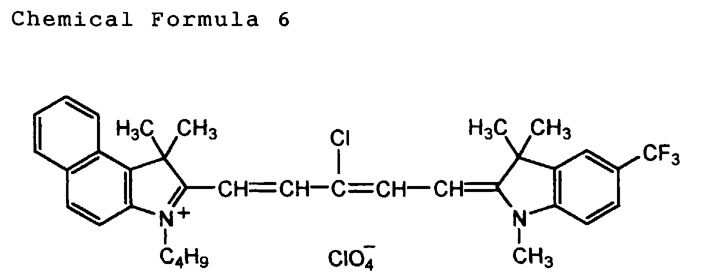

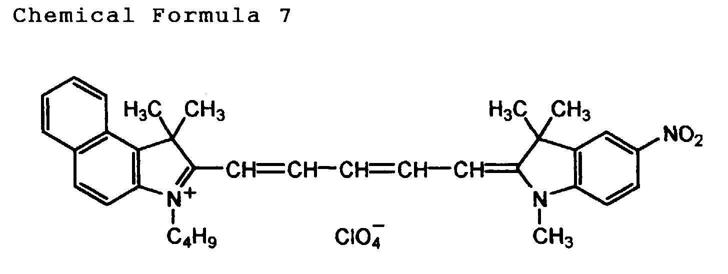

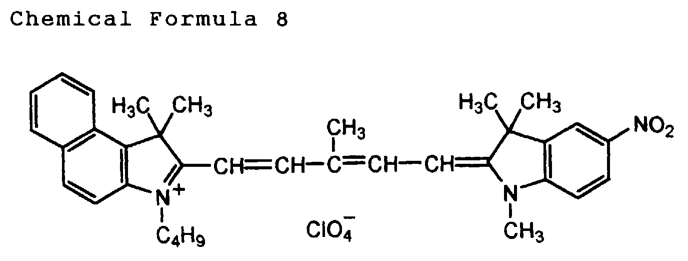

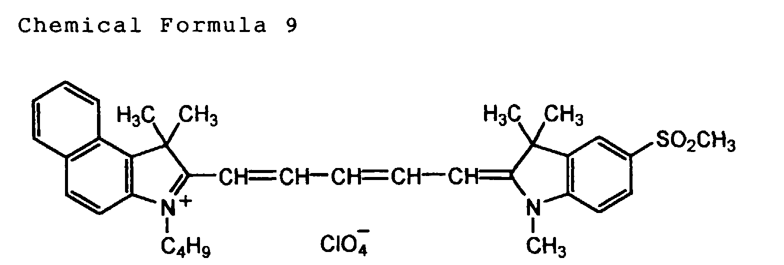

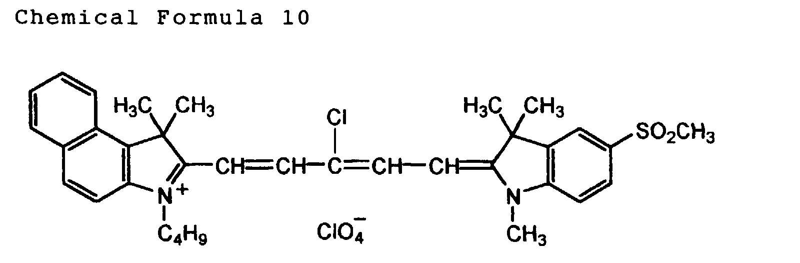

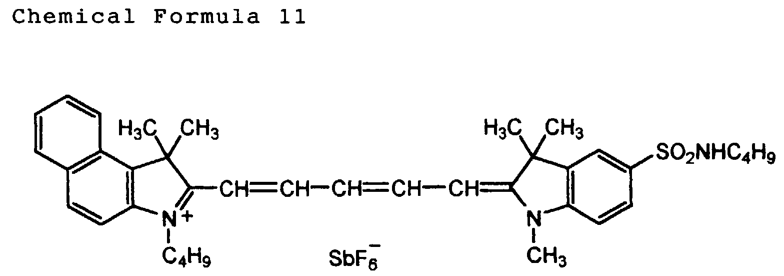

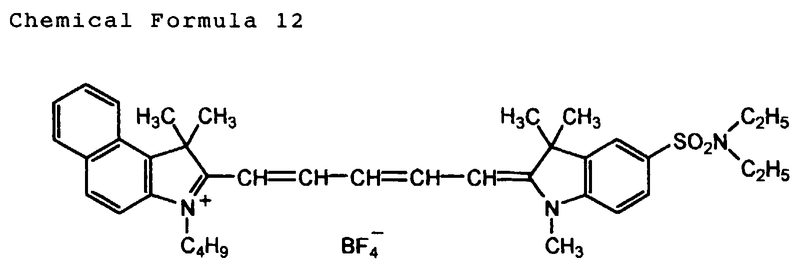

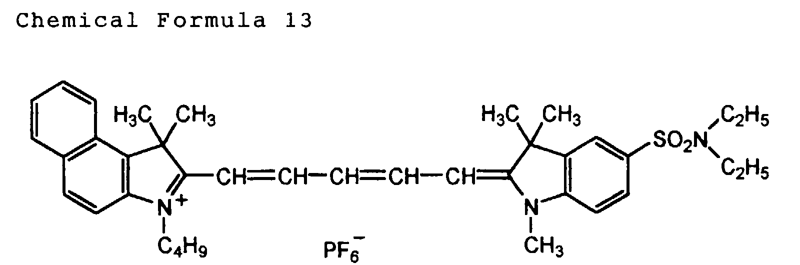

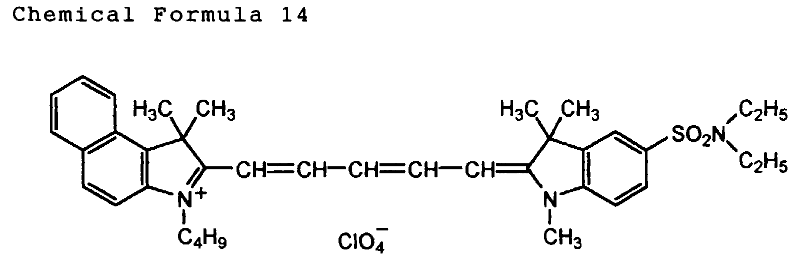

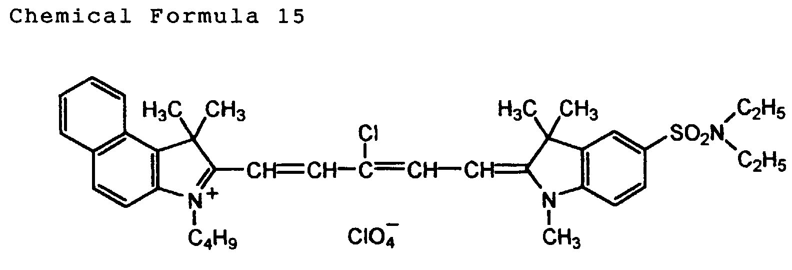

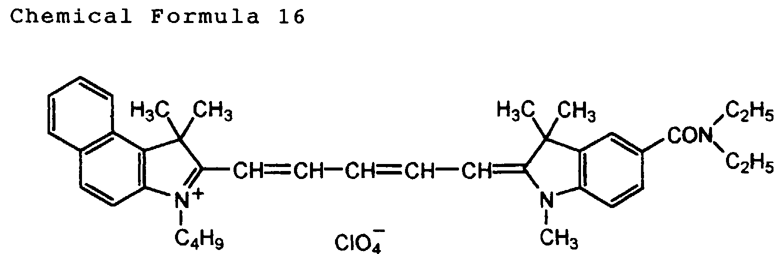

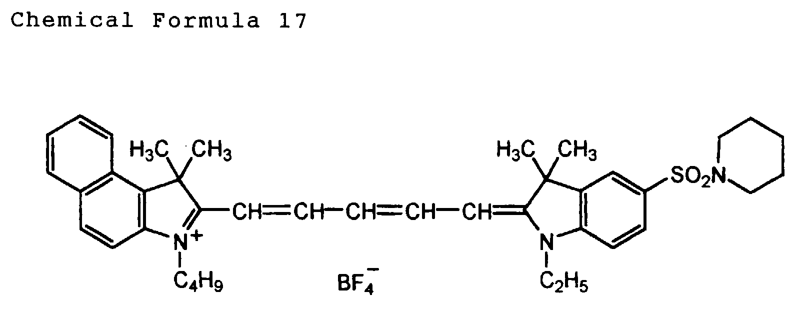

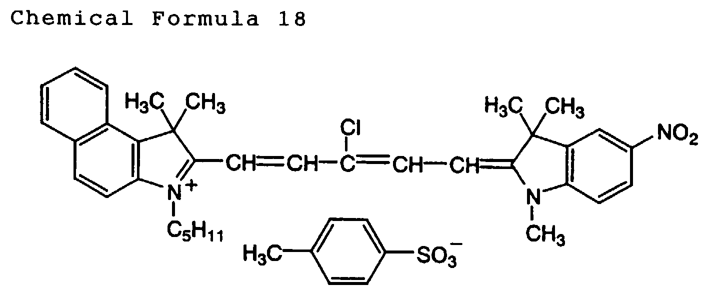

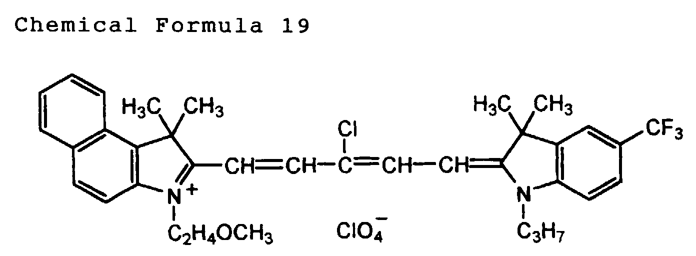

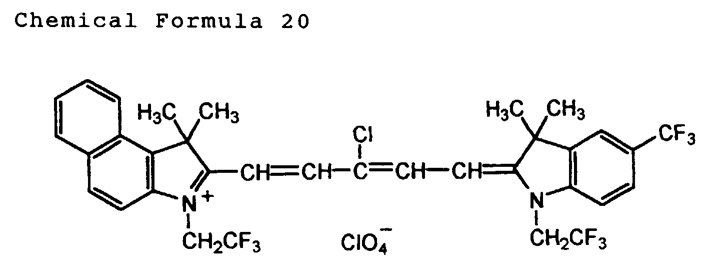

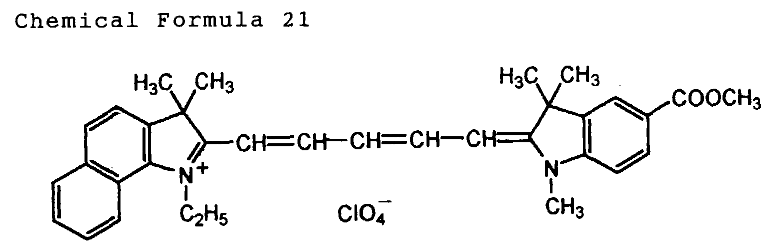

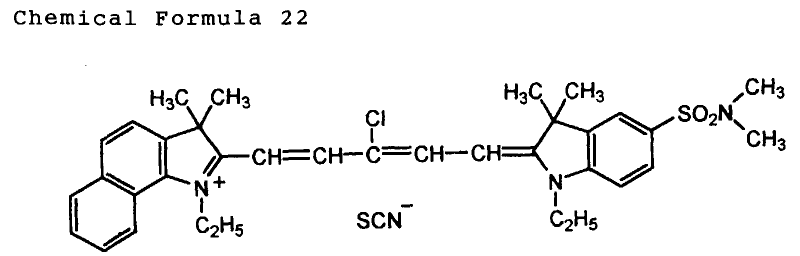

Although these cyanine dyes of the present invention can be prepared by various methods, they can be advantageously prepared through a step of reacting 3,3-dimethyl indolenium compounds having an active methyl group with 2-(1,3-butadienyl)-3,3-dimethyl indolenium compounds having an appropriate leaving group. With the method, the cyanine dyes of the present invention can be produced in a desired yield by reacting a compound, represented by Formula 3 or 4 where R₁ and R₄ correspond to those in Formula 1 or 2, with a compound, represented by Formula 5 where R₂ and R₃ correspond to those in Formula 1 or 2.

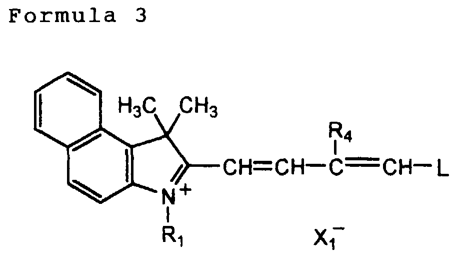

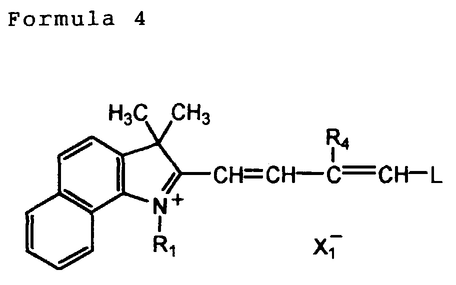

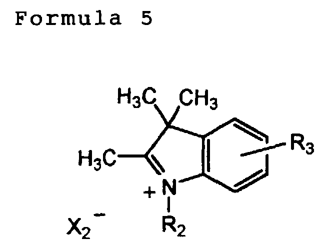

Appropriate amounts (usually about equal mols) of a compound represented by Formula 3 or 4 and a compound represented by Formula 5 are provided, and the resulting mixture is dissolved in an adequate solvent, and then reacted at ambient temperature or less than ambient temperature under heating and stirring conditions, for example a heating reflux, in the presence of a basic compound such as sodium hydroxide, potassium hidroxide, sodium acetate, potassium acetate, sodium carbonate, potassium carbonate, calcium carbonate, ammonia, triethylamine, piperidine, pyridine, pyrrolidine, morpholine, aniline, N,N-dimethylaniline, N,N-diethylaniline, or 1,8-diazabicyclo [5.4.0] -7-undecene; an acidic compound such as hydrochloric acid, sulfuric acid, nitric acid, methanesulforic acid, p-toluenesulfonic acid, acetic acid, propionic anhydride, trifluoroacetic acid, or trifluorosulfonic acid; or a Lewis acidic compounds such as aluminium chrolide, zinc chrolide, tin tetrachloride, or titanium tetrachloride.

As a solvent, the following can be used: Hydrocarbons such as pentane, hexane, cyclohexane, octane, benzene, toluene, and xylene; halogen compounds such as carbon tetrachloride, chloroform, 1,2-dichloroethane, 1,2-dibromoethane, trichloroethylene, tetrachloroethylene, chlorobenzene, bromobenzene, and α-dichlorobenzene; alcohols and phenols such as methanol, ethanol, 1-propanol, 2-propanol, 1-butanol, 2-butanol, isobutyl alcohol, isopentyl alcohol, cyclohexanol, ethylene glycol, diethylene glycol, triethylene glycol, propylene glycol, 2-methoxyethanol, 2-ethoxyethanol, phenol, benzyl alcohol, cresol, and glycerin; ethers such as diethyl ether, diisopropyl ether, tetrahydrofuran, tetrahydropyran, 1,4-dioxane, anisole, 1,2-dimethoxyethane, diethylene glycol dimethyl ether, dicyclohexyl-18-crown-6, methylcarbinol, and ethylcarbitol; ketones such as furfural, acetone, ethyl methyl ketone, and cyclohexanone; acids and acidic derivatives such as acetic acid, acetic anhydride, trichloroacetic acid, trifluoroacetic acid, propionic anhydride, ethyl acetate, butyl carbonate, ethylene carbonate, propylene carbonate, formamide, N-methylformamide, N,N-dimethylformamide, N,N-acetoamide, hexamethylphosphoric triamide, and phosphoric trimethyl; nitriles such as acetonitrile, propionitrile, succinonitrile, and benzonitrile; nitro compounds such as nitromethane and nitrobenzene; sulfur-containing compounds such as dimethylsulfoxide; and water. These solvents as mentioned above can be used in an appropriate combination, if necessary.

In general, in use of a solvent, the more the use of solvent is, the lower the reactivity is. On the other hand, the less the use of solvent is, the more the uniform heating and stirring becomes difficult and the more a side reaction causes easily. Thus, a volume of solvent is desirably up to 100 times, usually 5 to 50 times, of material compounds used by weight. The reaction finishes within 10 hours, usually 0.5-10 hours, depending on the kinds of material compounds and reaction conditions. The process of reaction can be monitored in conventional methods, for example thin-layer chromatography, gas chromatography, and high-performance liquid chromatography. Thereafter, the cyanine dyes of the present invention with desired counter ions can be obtained from the reaction mixture directly, and if necessary, after treated with conventional counter ion-exchange reaction. Thus, all the cyanine dyes represented by Formulae 1 to 22 can be easily obtained by the above method in a desired yield. In Formulae 3 to 5, X₁⁻ and X₂⁻ are identically or differently appropriate counter ions each other as well as X⁻ in Formulae 1 and 2, and L is an appropriate leaving group which is usually selected from monovalent groups of aniline or aniline derivatives such as anilino, p-toluidino, p-methoxyanilino, p-ethoxycarbonylanilino, or N-acetylanilino group. All the compounds represented by Formula 3 or 5 can be prepared for example by a method disclosed in Japanese Patent kokai No. 316,655/98 applied for by the present applicant.

The cyanine dyes thus obtained can be used in the form of a reaction mixture without any further treatment, and usually used after purified by the following conventional methods, which are generally used for purifying their related compounds; dissolution, extraction, separation, decantation, filtration, concentration, thin-layer chromatography, column chromatography, gas chromatography, high-performance liquid chromatography, distillation, crystallization, and sublimation. If necessary, two or more of these methods can be used in combination. For use as a light absorbent in optical recording media such as CD-Rs, etc., the cyanine dyes of the present invention should preferably be distilled, crystallized, and/or sublimated prior to use.

Light absorbents of the present invention comprise any one of the cyanine dyes and are to utilize the nature of such cyanine dyes which substantially absorb visible light. The composition or the physical form of the light absorbents is not restricted. The light absorbents of the present invention comprise the cyanine dye represented by Formulae 1 or 2, and additionally contain one or more the other elements depending on use. One of the fields, which light absorbents of the present invention can be advantageously used, is of optical recording media. In this field, the light absorbents of the present invention are suitable as a material for recording layer inorganic optical recording media, and particularly optical recording media using laser beams with wavelengths of around 780 nm as a writing light, and usually CD-Rs using laser beams with wavelengths of 775-795 nm. In the case of using the light absorbents of the present invention for optical recording media, unless the use deviates from the object of the present invention, the cyanine dyes represented by Formula 1 or 2 can be mixed with other light absorbents comprising other dye compounds which are sensitive to visible light, and one or more conventional materials in optical recording media such as light-resistant improves, binders, dispersing agents, flame retardants, lubricants, antistatic agents, surfactants, and plasticizers, if necessary.

Explaining the uses of the light absorbents of the present invention, for example, optical recording media, the cyanine dyes of the present invention have absorption maxima in visible region and substantially absorb laser beams with wavelengths of around 780 nm, when formed in a thin layer. When most of them are measured by the method of the later described Example, they have decomposition points (exothermic starting temperatures in differential thermal analysis), which are closely adjacent (i.e., usually within 10°C or narrower) to their melting points (endothermic starting temperature); have only decomposition points or those which are easily undistinguishable from their melting points; have decomposition points of not lower than 240°C, and usually not lower than 260 °C; and promptly decompose at around their decomposition points. Accordingly, unlike conventional related compounds, when the cyanine dyes of the present invention are used as a light absorbent in high-speed-recordable-type optical recording media such as CD-Rs, only cyanine dyes positioning at the irradiated points promptly decompose and form prescribed pits without deforming the previously-formed pits nearness to the newly-formed pits by the conduction of heat of fusion and decomposition to the part around the irradiated points. Accordingly, prescribed pits can be promptly formed on the limited recording surface of the optical recording media. Cyanine dyes, which have relatively-low melting points and decomposition points, have the drawback that they tend to deform the part around the pits, which have been previously-formed on the recording surface, and other pit-less part by the accumulated heat generated when optical recording media are irradiated by reading light for reading informations for a relatively-long period of time, and that the dyes *per se* may be deteriorated. However, the cyanine dyes of the present invention substantially do not cause such a problem because they have only decomposition points or those which are easily undistinguishable from the melting points, and because the decomposition points are relatively high. Furthermore, most of the cyanine dyes have a satisfactory-high solubility in organic solvents such as TFP, which are commonly used in the field of optical recording media, and this easily increases the product yield and the working efficiency for coating cyanine dyes as a light absorbent over substances for optical recording media, and maintains the product quality and property at a relatively-high level.

Optical recording media of the present invention can be prepared in accordance with the processes for conventional ones because the cyanine dyes of the present invention do not require any special treatment and step when used in optical recording media. For example, as a light absorbent, one or more of the cyanine dyes of the present invention can be mixed with one or more other dye compounds which are sensitive to visible light, and one or more conventional light-resistant improvers, binders, dispersing agents, flame retardants, lubricants, antistatic agents, surfactants, and plasticizers to control the reflection percentage and the light absorption percentage in recording layers, if necessary. The resulting mixture is dissolved in an organic solvent, and the solution is uniformly coated over either surface of substrates in such a manner of using spraying, soaking, roller coating, or rotatory coating method; and dried to form a thin layer as a recording layer, and if necessary, followed by forming a reflection layer to be closely attached onto the recording layer by the method of vacuum deposition, chemical vapor deposition, sputtering, or ion plating using metals such as gold, silver, copper, platinum, aluminium, cobalt, tin, nickel, iron, and chromium to impart a reflection efficiency of 65% or more, and preferably 75% or more; forming the reflection layer to be closely attached onto the recording layer by using conventional organic materials for reflection layers; and spun coating over the recording layer ultraviolet-ray-hardening resins or thermosetting resins, which contain flame retardants, stabilizers, and/or antistatic agents, and then hardening the coated resins by either irradiating light or heating to form a protection layer to be closely attached onto the reflection layer to protect the recording layer from scratches, dusts, spoils, etc.

As other dye compounds usable in combination with the present cyanine dyes, all dye compounds can be used as long as they are sensitive to visible light and can regulate a light reflection rate and a light absorption rate of a recording layer in an optical recording media. As the dye compounds, the following compounds can be used in an appropriate combination, if necessary: Acridine dye, azaannulene dye, azo metal complex dye, anthraquinone dye, indigo dye, indanthrene dye, oxazine dye, xanthene dye, dioxazine dye, thiazine dye, thioindigo dye, tetrapyrapolphyradine dye, triphenylmethane dye, triphenylthiazine dye, naphthoquinone dye, phthalocyanine dye, benzoquinone dye, benzopyran dye, benzofuranone dye, porphyrin dye, rhodamine dye, and polymethine dye such as cyanine dye, merocyanine dye, oxanole dye, azulenium dye, squalilium dye, pyririum dye, thiopyririum dye, and phenanthrene dye which same or different rings are bound to the both ends of polymethine chain such as monomethine, dimethine, trimethine, tetramethine, pentamethine, hexamethine, or heptamethine. The chain and rings can have one or more substituents. As a ring, imidazolin ring, imidazole ring, banzoimidazole ring, α-naphthimidazole ring, β-naphthimidazole ring, indole ring, isoindole ring, indolenine ring, isoindolenine ring, benzoindolenine ring, pyridinoindolenine ring, oxazoline ring, oxazole ring, isooxazole ring, benzooxazole ring, pyridinooxazole ring, α -naphthoxazole ring, β-naphthoxazole ring, selenazoline ring, selenazole ring, benzoselenazole ring, α -naphthselenazole ring, β -naphthselenazole ring, thiazoline ring, thiazole ring, isothiazole ring, benzothiazole ring, α-naphththiazole ring, β-naphththiazole ring, tellurazoline ring, tellurazole ring, benzotellurazole ring, α -naphthtellurazole ring, β-naphthtellurazole ring, acridine ring, anthracene ring, isoquinoline ring, isopyrrole ring, imidanoxaline ring, indandione ring, indazole ring, indaline ring, oxadiazole ring, carbazole ring, xanthine ring, quinazoline ring, quinoxaline ring, quinoline ring, chroman ring, cyclohexanedion ring, cyclopentandion ring, cinnoline ring, thiodiazole ring, thiooxazolidone ring, thiophene, thionaphthene, thiobarbituric acid ring, thiohydantoin ring, tetrazole ring, triazine ring, naphthalene ring, naphthyridine ring, piperazine ring, pyrazine ring, pyrazole ring, pyrazoline ring, pyrazolidine ring, pyrazolone ring, pyran ring, pyridine ring, pyridazine ring, pyrimidine ring, pyrylium ring, pyrrolidine ring, pyrroline ring, pyrrole ring, phenazine ring, phenanthridine ring, phenanthrene ring, phenanthroline ring, phthalazine ring, pteridine, furazane ring, furan ring, purine ring, benzene ring, benzoxazine ring, benzopyran ring, morpholine ring, and rhodanine ring. As the dye compounds used in combination with the present cyanine dye, they are desirably to have the absorption maxima in visible region, particularly, at wavelengths of 400-850 nm, when in a thin layer.

The light-resistance improvers used in the present invention are, for example, nitroso compounds such as nitrosodiphenylamine, nitrosoaniline, nitrosophenol, and nitrosonaphthol and metal complexes such as tetracyanoquinodimethane compounds, diimmonium salt, and "NKX-1199" (bis[2'-chloro-3-methoxy-4-(2-methoxyethoxy)dithiobenzyl] nickel) produced by Hayashibara Biochemical Laboratories, Inc., Okayama, Japan, and formazane metal complexes, which all can be appropriately used in combination, if necessary. Preferable light-resistance improvers are those which contain nitroso compounds or formazane metal compounds, and most preferable ones are nitroso compounds, which have a phenylpyridylamine skeleton as disclosed in Japanese Patent Application No. 88,983/99, titled "Phenylpyridylamine derivatives" applied for by the present applicant; formazane compounds, which have a pyridine-ring at C-5 and a pyridine- or furan-ring at C-3 of formazane skeleton as disclosed in Japanese Patent Application No. 163,036/99, titled "Formazane metal complexes" applied for by the present applicant; and complexes to metals such as nickel, zinc, cobalt, iron, copper, and palladium, which have one or more tautomers of the aforesaid compounds as a ligand. In the case of using such a light-resistance improver in combination, the cyanine dyes of the present invention can be effectively prevented from undesirable changing in deterioration, fading, color changing, and quality changing, which are inducible by environmental-lights such reading- and natural-lights, without lowering the solubility of the cyanine dyes in organic solvents and substantially deteriorating preferable optical features. As a composition ratio, 0.01-5 moles, and preferably 0.1-1 moles of a light-resistance improver(s) can be incorporated into one mole of the present cyanine dye(s) while increasing or decreasing the ratio.

The cyanine dyes of the present invention have a satisfactory-high solubility in organic solvents without substantially causing negative problem for actual use, and do not substantially restrict organic solvents used for coating the cyanine dyes on substrates. Thus, in the preparation of optical recording media of the present invention, appropriate organic solvents can be selected from the following and appropriately used in combination: TFP frequently used to prepare optical recording media and the following organic solvents other than TFP: For example, hydrocarbons such as hexane, cyclohexane, methylcyclolhexane, dimethylcyclohexane, ethylcyclohexane, isopropylcyclohexane, tertbutylcyclohexane, octane, cyclooctane, benzene, toluene, and xylene; halogen compounds such as carbon tetrachloride, chloroform, 1,2-dichloroethane, 1,2-dibromoethane, trichloroethylene, tetrachloroethylene, chlorobenzene, bromobenzene, and α -dichlorobenzene; alcohols and phenols such as methanol, ethanol, 2,2,2-trifluoroethanol, 2-methoxyethanol (methyl cellosolve), 2-ethoxyethanol (ethyl cellosolve), 2-isopropoxy-1-ethanol, 1-propanol, 2-propanol, 1-methoxy-2-propanol, 1-ethoxy-propanol, 1-butanol, 1-methxy-2-butanol, 3-methoxy-1-butanol, 4-methoxy-1-butanol, isobutyl alcohol, isopentyl alcohol, cyclohexanol, ethylene glycol, propylene glycol, phenol, benzyl alcohol, cresol, diethylene glycol, triethylene glycol, glycerine and diacetone alcohol; ethers such as diethyl ether, diisopropyl ether, tetrahydrofuran, tetrahydropyran, 1,4-dioxane, anisole, 1,2-dimethoxyethane, diethylene glycol dimethyl ether, cyclohexyl-18-crown-6, methylcarbinol, and ethylcarbitol; ketones such as furfural, acetone, 1,3-diacetyl acetone, ethyl methyl ketone, and cyclohexanone; esters such as ethyl acetate, butyl acetate, ethylene carbonate, propylene carbonate, and trimethyl phosphate; amides such as formamide, N-methyl formamide, N,N-dimethylformamide, and hexamethylphosphoric triamide; nitriles such as acetonitrile, propionitrile, and succinonitrile; nitro compounds such as nitromethane and nitrobenzene; amines such as ethylene diamine, pyridine, piperidine, morpholine, and N-methylpyrrolidone; and sulfur-containing compounds such as dimethylsulfoxide and sulfolane. These organic solvents as mentioned above can be used in an appropriate combination.

Particularly, the cyanine dyes of the present invention have a relatively-high solubility in easily-volatile organic solvents such as TFP, methyl cellosolve, ethyl cellosolve, and diacetone alcohol, and thus they are substantially free of crystallization when dissolved successively in the organic solvents, spun coated on substrates, and dried without substantially causing dye crystals and inconsistency of the thickness and the surface of the layers formed on optical recording media. Most of the cyanine dyes of the present invention well dissolve in non-halogen solvents, for example, alcohols such as methyl cellosolve and ethyl cellosolve; and ketones such as diacetone alcohol and cyclohexanone. The use of such solvents has the merit that the solvents are free from damaging the substrates and spoiling the environment, when the cyanine dyes of the present invention dissolve in the alcohols before coated on the substrates.

Conventional substrates can be used in the present invention. The substrates used in the present invention can be usually processed with appropriate materials, for example, into discs, 12 cm in diameter and 0.6-1.2 mm in thickness, to suite to final use by the methods such as compression molding, injection molding, compression-injection molding, photopolymerization method (2P method), thermosetting integral method, and lightsetting integral method. Such discs can be used in single or plural after appropriately attaching them together with adhesive sheets or adhesive agents, etc. In principal, any materials for substrates can be used in the present invention as long as they are substantially transparent and have a transmissivity of at least 80%, and preferably at least 90% or more at wavelengths ranging from 400 nm to 850 nm. Examples of such materials are glasses, ceramics, and others such as plastics such as polyacrylate, polymethyl methacrylate, polycarbonate, polystyrene (styrene copolymer), polymethylpenten, polyester, polyolefin, polyimide, polyetherimide, polysulfone, polyethersulfone, polyarylate, polycarbonate/polystyrene alloy, polyestercarbonate, polyphthalatecarbonate, polycarbonateacrylate, non-crystalline polyolefin, methacrylate copolymer, diallylcarbonatediethylene-glycol, epoxy resin, and phenolic resin, where polycarbonate is most frequently used. In plastic substrates, concaves for expression of synchronizing signals and addresses of tracks and sectors are usually transferred to the internal circle of the tracks during their formation. The form of concaves is not specifically restricted and is preferably formed to give 0.3-1.2 µm in average wide and 70-200 nm in width.

The light absorbents of the present invention can be prepared into 0.5-5 w/w % solutions of the organic solvents as mentioned above while considering the viscosity in the solution, and then uniformly coated onto a substrate to form a dried recording layer with 10-1,000 nm, and preferably 50-300 nm in thickness. Prior to the coating, a precoating layer can be formed over the substrate to improve the protection and the adhesion-ability to the substrate, if necessary. Materials of the precoating layer are, for example, high-molecular substances such as ionomer resins, polyamide resins, vinyl resins, natural resins, silicon, and liquid rubbers. In the case of using binders, the following polymers can be used alone or in combination in a weight ratio of 0.01-10 times of the cyanine dye(s): Cellulose esters such as nitrocellulose, cellulose phosphate, cellulose sulfate, cellulose acetate, cellulose propionate, cellulose lactate, cellulose palmitate, and cellulose acetate/propionate; cellulose ethers such as methyl cellulose, ethyl cellulose, propyl cellulose, and butyl cellulose; vinyl resins such as polystyrene, poly(vinyl chloride), poly(vinyl acetate), poly(vinyl acetal), poly(vinyl butyral), poly(vinyl formal), poly(vinyl alcohol), and poly(vinyl pyrrolidone); copolymer resins such as styrene-butadiene copolymers, styrene-acrylonitrile copolymers, styrene-butadiene-acrylonitrile copolymers, vinyl chloride-vinyl acetate copolymers, and maleic anhydride copolymers; acrylic resins such as poly(methyl methacrylate), poly(methyl acrylate), polyacrylate, polymethacrylate, polyacrylamide, and polyacrylonitrile; polyesters such as poly(ethylene terephthalate); and polyolefins such as polyethylene, chlorinated polyethylene, and polypropylene.

Explaining the method for using the optical recording media according to the present invention, the high-speed-writable-type optical recording media of the present invention such as CD-Rs are writable informations at a higher-speed than a normal linear velocity of CD-Rs (1.2 to 1.4 m/sec) by using laser devices, for example, with oscillating wavelengths at around 775-795 nm, irradiated by semiconductor lasers such as those of AlGaInP, GaAsP, GaAlAs, InGaP, InGaAsP or InGaAlP; or YAG lasers combined with second harmonic generation inducing element (SHG element). To read recorded informations, laser beams are used with wavelengths identical to or slightly longer than those used for writing informations, for example, those of 770-830 nm. As for the laser power for writing and reading informations, in the optical recording media of the present invention, it is preferably be set to a relatively-high level which exceeds the threshold of the energy required for forming pits when used for writing information, while it is suitably be set to a relatively-low level, i.e., a level of below the threshold when used for reading the recorded informations, although the power levels can be varied depending on the types and ratios of the light-resistance improvers used in combination with the cyanine dyes: Generally, the levels can be controlled to powers at least 5 mW, and usually 10-50 mW for writing; and to powers of 0.1-5 mW for reading. The recorded informations are read by detecting the changes of both the reflection light level and the transmission light level in the pits and the pit-less part on the surface of optical recording media according to the method of light pick-up.

Since the optical recording media of the present invention can record informations of characters, images, voices and other digital informations at a high-density, they are extremely useful as recording media for professional and family use to record and keep documents, data, and computer softwares. Examples of the types of industries and forms of informations, to which the optical recording media can be applied, are as follows: Drawings of constructions and engineering works, maps, ledgers of loads and rivers, aperture cards, architectural sketches, documents of disaster protection, wiring diagrams, arrangement plans, informations of news papers and magazines, local information, and construction specifications, which all relate to constructions and engineering works; blueprints, ingredient tables, prescriptions, product specifications, product price tables, part's lists, information for maintenance, case study files of accidents and troubles, manuals for claims, production schemes, technical documents, sketches, details, company's house-made product files, technical reports, and analysis reports, which are all used in productions; customer's informations, informations of business connections and companies, contracts, informations of newspapers and magazines, business reports, company's inquires into the financial status, and stock lists, which are all used for sales; information of companies, records of stock prices, statistical documents, informations of newspaper and magazines, contracts, customer's lists, documents of application/notification/licenses/authorization, and business reports, which are all used in finance business; information of real property, sketches of constructions, maps, and local information, informations of newspaper and maps, contracts for lease, informations of companies, stock lists, traffic informations, and informations of business connections, which are all used for real property and transportations; diagrams of writings and pipe arrangements for electric and gas supplies, documents of disaster protection, tables of operation manuals, documents of investigations, and technical reports; medical cartes, files of clinical histories and case studies, and diagrams for medical care/institution relationships; texts, collections of questions, educational documents, and statistical information, which are all used in private and preparatory schools; scientific papers, records in academic societies, monthly reports of researches, data of researches, documentary records and indexes thereof, which are all used in universities, colleges, and research institutes; inspection data, literatures, patent publications, weather maps, analytical records of data, and customer's files, which are all used for information; case studies on laws; membership lists, history notes, records of works/products, competition data, and data of meetings/congresses, which organizations/associations; sightseeing information and traffic information, which are all used for sightseeing; indexes of homemade publications, information of news papers and magazines, who's who files, sport records, telop files, and scripts, which are all used in mass communications and publishers; and maps, ledgers of roads and livers, fingerprint files, resident cards, documents of application/notification/license/authorization, statistical documents, and public documents, which are all used in government offices. Particularly, the write-once type optical recording media of the present invention can be advantageously useful for storing records of cartes and official documents, and electric libraries for art galleries, libraries, museums, broadcasting stations, etc.

As a rather specific use, the optical recording media of the present invention can be used to edit compact discs, digital video discs, laser discs, MD (a mini disc as an information recording system using a photomagnetic disc), CDV (a laser disc using compact disc), DAT (an information recording system using magnetic tape), CD-ROM (a read-only memory using compact disc), DVD-ROM (a read-only memory using digital video disc), DVD-RAM (a writable and readable memory using digital video disc), digital photos, movies, video softwares, audio softwares, computer graphics, publishing products, broadcasting programs, commercial messages, game softwares, etc.; and used as external program recording means for large size of computers and car navigation systems.

Hereinbefore described are the application examples of the cyanine dyes of the present invention to organic optical recording media which use laser beams with wavelengths of around 780 nm as a wrighting light. However, in the field of optical recording media, the cyanine dyes of the present invention can be also used to regulate or correct the optical absorption rate or optical reflection rate in the optical recording media such as CD-R and high-density optical recording media such as DVD-R by using in combination, for example, with one or more other organic dye compounds which substantially absorb laser beams with wavelengths of around 650 nm. Further, the cyanine dyes of the present invention can be used to form pits on substrates in the media in such a manner that an excitation energy by laser beams with wavelengths of around 650 nm transfers to the cyanine dyes through the above organic dye compounds. The optical recording media of the present invention mean all optical recording media in general which utilize the characteristics of the cyanine dyes of the present invention that substantially absorb visible light. In addition to organic optical recording media, thermal coloration method using a chemical reaction of coloring agents and developers using the heat is generated when organic dye compounds absorb light, and the technique called "moth-eye type technique" which uses a phenomenon that the above heat smooths the pattern of periodical unevenness provided on the surface of the substrates.

Since the cyanine dyes of the present invention have each absorption maxima in visible region and substantially absorb visible lights, the light absorbents of the present invention containing the cyanine dyes are very useful as materials to polymerize polymerizable compounds by exposing visible lights, to photosensitize solar batteries, and to dye clothes, in addition to the uses in optical recording media. If necessary, in combination with one or more other light absorbents capable of absorbing light in ultraviolet, visible and/or a infrared region, the light absorbents can be used in clothes in general and others including building/bedding/decorating/products such as a drape, lace, casement, print, casement cloth, roll screen, shutter, shop curtain, blanket, thick bedquilt, including comforter, peripheral material for the thick bedquilt, cover for the thick bedquilt, cotton for the thick bedquilt, bed sheet, japanese cushion, pillow, pillow cover, cushion, mat, carpet, sleeping bag, tent, interior finishing for car, and window glasses including car window glass; sanitary and health goods such as a paper diaper, diaper cover, eyeglasses, monocle, and lorgnette; internal base sheets, linings, and materials for bags; wrappers; materials for umbrellas; parasols; stuffed toys; lighting devices; filters, panels and screens for information displaying devices such as cathode-ray tubes, liquid crystal displays, electrolytic luminous displays, and plasma displays; sunglasses; sunroofs; sun visors; pet bottles; refrigerators; vinyl houses; lawns; optical fibers; prepaid cards; and peeping windows in electric ovens, and other type ovens. When used as wrapping materials, injection materials, and vessels for the above products, the light absorbents of the present invention prevent living bodies and products from the troubles and discomforts caused by environmental light such as natural and artificial light or even lower the troubles and discomforts, and furthermore they can advantageously regulate the color, tint, and appearance and control the light reflected by or passed the products to a desired color balance.

The following examples describe the preferred embodiments of the present invention:

### Example 1

### Cyanine dye

In a reaction vessel, 20 g of 1-butyl-2-[4-(N-acetylphenylamino)-1,3-butadienyl]-3,3-dimethylbenzoindolenium=perchlorate, and 16 g of 1,2,3,3-tetramethyl-5-nitroindolenium=tosylate were placed and then admixed with 80 ml of methanol and 8 ml of triethylamine. The mixture was reacted at 60 °C for one hour under stirring conditions. The reaction mixture was cooled to ambient temperature and appropriately admixed with acetone to disperse oily matters. The resulting crystallized crude crystal was collected by filtration and recrystallized in a mixture of methanol and chloroform to obtain 3.91 g of a golden brown crystal of the cyanine dye represented by Chemical Formula 7.

The cyanine dye of this Example has various uses as a light absorbent in many fields including those of optical recording media.

### Example 2

### Cyanine dye

In a reaction vessel, 10 g of 1-butyl-2-[4-(N-acetylphenylamino)-1,3-butadienyl]-3,3-dimethylbenzoindolenium=perchlorate, and 9.7 g of 1,2,3,3-tetramethyl-5-diethylaminosulfonylindolenium=perchlorate were placed and then admixed with 40 ml of ethanol and 3.9 ml of triethylamine. The mixture was reacted at 50 °C for 45 minutes under stirring conditions. The reaction mixture was cooled to ambient temperature and appropriately admixed with ethanol to disperse oily matters. The resulting crystallized crude crystal was collected by filtration and recrystallized in a mixture of methanol and acetone to obtain 9.2 g of a bluish green crystal of the cyanine dye represented by Chemical Formula 14.

The cyanine dye of this Example has various uses as a light absorbent in many fields including those of optical recording media.

Although the production yield and conditions are varied in some degrees depending on the structures of the cyanine dyes of the present invention, all the cyanine dyes of the present invention, including the compounds represented by Chemical Formulae 1 to 22, can be produced by the methods in Examples 1 and 2 and in accordance therewith.

### Example 3

### Light feature of cyanine dye

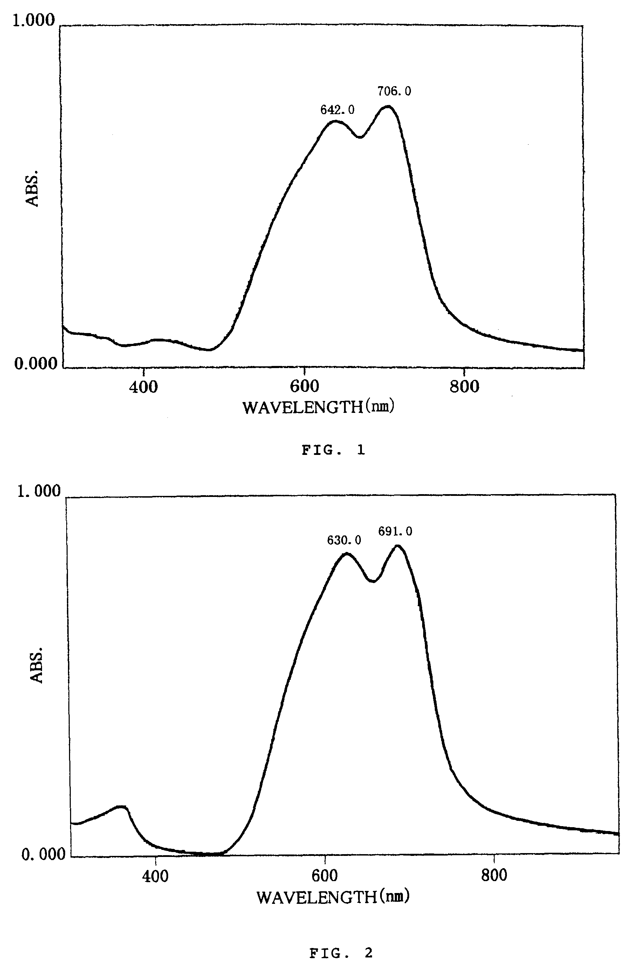

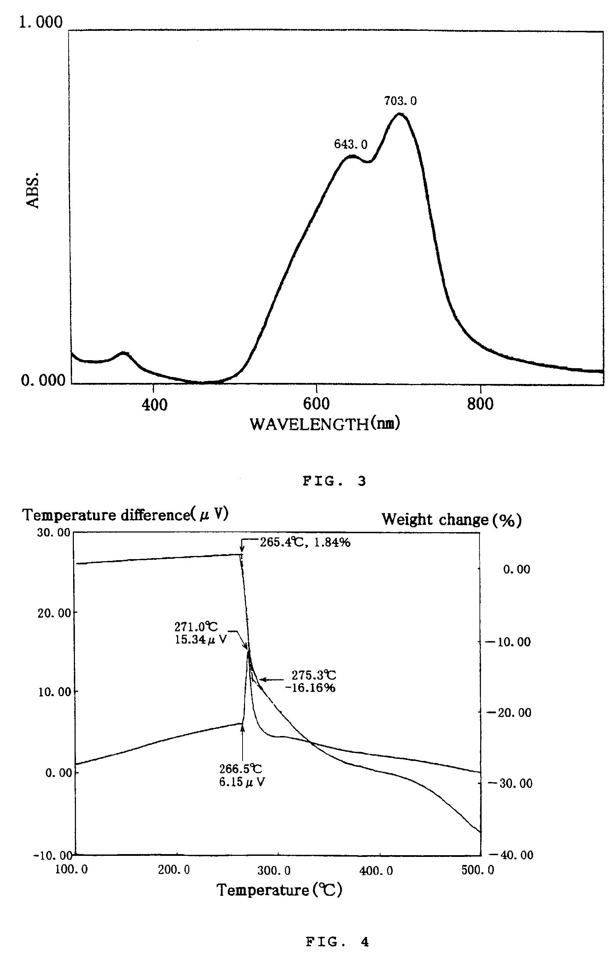

An adequate amount of crystal of each cyanine dyes of the present invention, as shown in Table 1, were measured in a conventional manner for visible absorption spectrum when in a methanol solution, respectively. In parallel, a known related compound represented by Chemical Formula 23 was similarly measured for visible absorption spectrum. Table 1 shows the absorption maximum wavelengths for each cyanine dyes, respectively, when formed in a solution. FIGs. 1, 2 and 3 show the visible absorption spectra for the cyanine dyes represented by Chemical Formulae 7, 14 and 23, respectively. They were measured in a conventional manner when formed in a layer over a glass substrate. A cyanine dye represented by Chemical Formula 23 is a related compound which is useful as a light absorbent in usual CD-Rs.

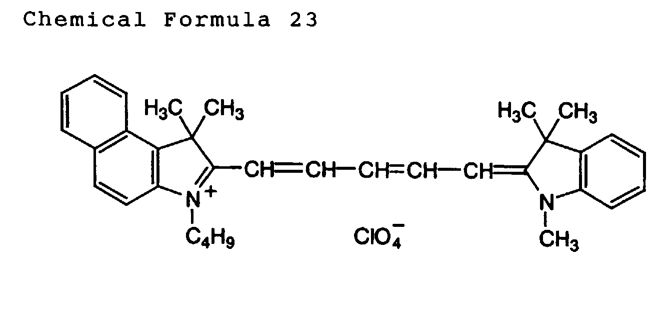

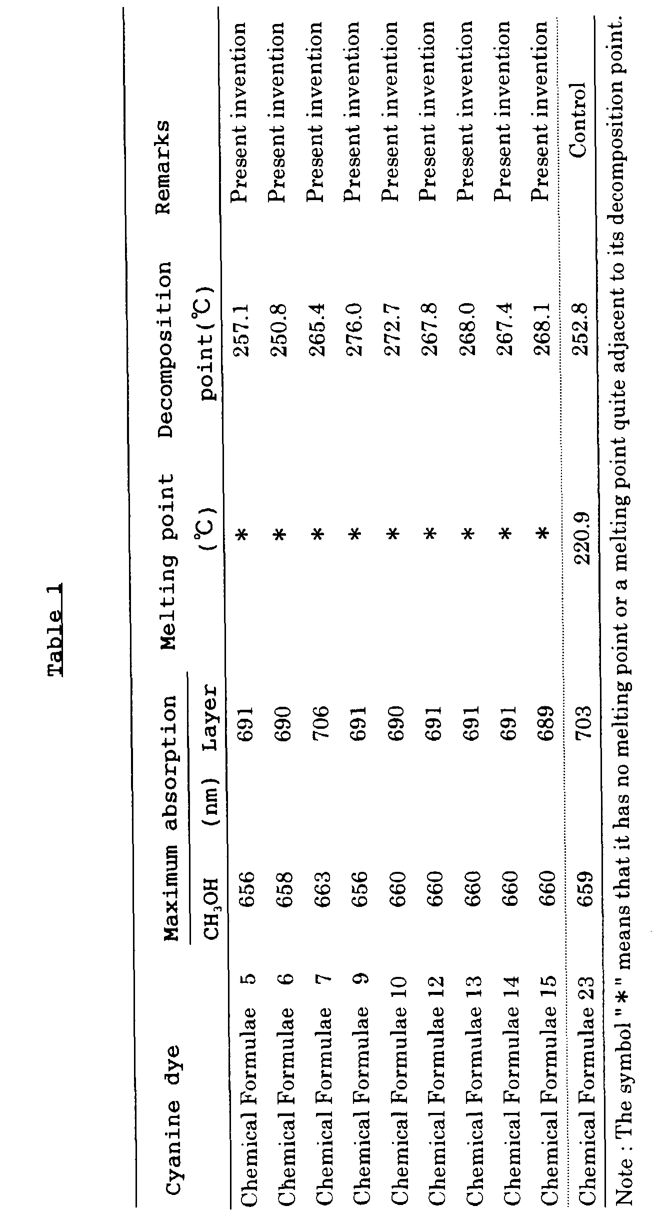

As shown in the results in Table. 1, all of the measured cyanine dyes of the present invention had each absorption maxima in visible region with wavelengths of 650-670 nm which were similar to that of the known related compound represented by Chemical Formula 23, when formed in a solution. As shown in the visible absorption spectra in FIGs.1 to 3, the cyanine dyes represented by Chemical Formulae 7 and 14 had the spectra which were similar to that of the known related compound represented by Chemical Formula 23, and they had the absorption maxima in visible region with wavelengths of 690-710 nm, when formed in a solution, and the absorption end to the side of longer-wavelength area extended to around 850 nm. These results show that all the cyanine dyes of the present invention including the dyes represented by Chemical Formulae 7 and 14 have the spectra which are similar to that of the known related compound represented by Chemical Formula 23, and substantially absorb laser beams with wavelengths of around 780 nm.

### Example 4

### Thermal property of cyanine dye

An adequate amount of. either the cyanine dyes of the present invention represented in Table 1, as a test specimen, was subjected to conventional differential thermal analysis (hereinafter abbreviated as "DTA") and thermogravimetric analysis (hereinafter abbreviated as "TGA") using "MODEL TG/DTA 220", a digital thermo analyzer commercialized by Seiko Instruments Inc., Tokyo, Japan, and was measured for their melting points and decomposition points. Table 1 shows their results. In parallel, a known cyanine dye represented by Chemical Formula 23 was analyzed similarly as above. FIGs. 4, 5 and 6 show the results of DTA and TGA of the cyanine dyes represented by each Chemical Formula 7, 14 and 23, respectively. In FIGs. 4 to 6, the solid line shows a change on temperature variation (µV) and the broken line shows a weight change (%). In DTA and TGA, the environmental temperature was set to an increasing temperature mode at a rate of 10 °C/min.

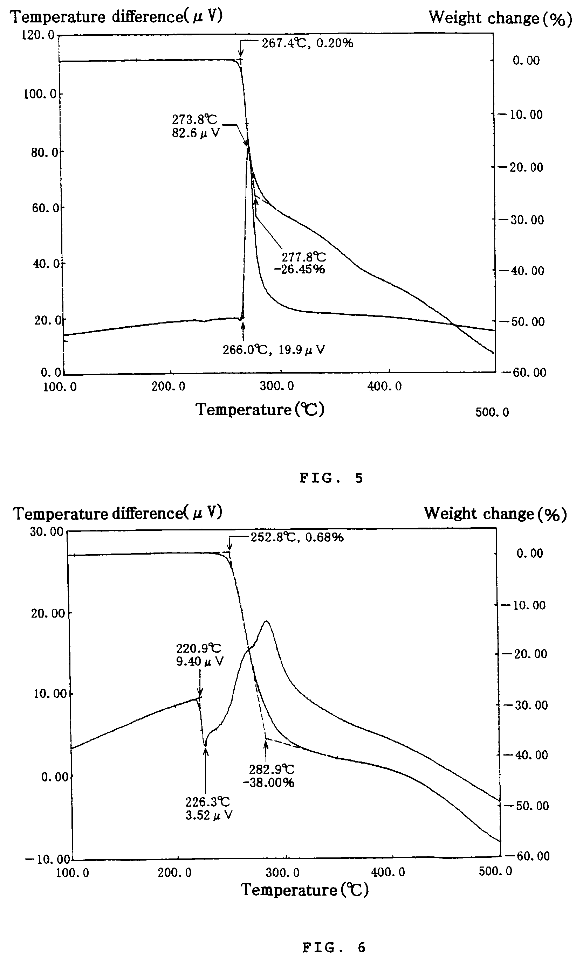

As shown in FIG. 6, a known related compound represented by Chemical Formula 23 had a melting point and a decomposition point at around 221°C and 253°C, respectively, and they were different from each other and the difference in temperature widened up to about 30°C. On the other hand, as found in Table 1, FIGs. 4 and 5, the measured cyanine dyes of the present invention had only decomposition points or one that were not easily distinguishable from the melting points. The decomposition points were equal to that of the known related compound represented by Chemical Formula 23 (about 240-260°C ), or were significantly higher than that of the known related compound (not lower than 260°C). As shown in Fig. 6, the known related compound represented by Chemical Formula 23 started to decompose at around 253°C and terminated to melt at around 283 °C, and the difference in temperature was 30 °C. As shown in FIGs. 4 and 5, in the cyanine dyes of the present invention represented by Chemical Formulae 7 and 14, the differences in temperature from starting to terminating of their decomposition were 10°C or narrower, and this showed the fact that the cyanine dyes of the present invention decomposed promptly at around their decomposition points.

As a cyanine dye represented by Chemical Formula 23, a dye slowly decomposed is difficult to form desirable pits on a limited recording layer quickly when used in high-speed-recordable-type optical recording media such as CD-Rs. Dyes with lower decomposition points can be generally used to write informations by using lower-power laser beams as a merit, however, as a demerit, when exposed to laser beams for a relatively-long period of time on reading, the dyes tend to accumulate heat and deform the parts around the pits and other pit-less parts formed on recording surfaces, resulting in large jitters and reading errors. When formed in a thin layer, the cyanine dyes of the present invention substantially absorb laser beams with wavelengths of 780 nm or shorter, have decomposition points which are undistinguishable from their melting points or have only decomposition points of relatively-high temperatures, and have an outstandingly-high decomposition rate at around the decomposition points. These demonstrate that optical recording media, having a relatively-small jitter, insubstantial reading error, and satisfactory stability to the exposure to environmental lights such as reading and natural lights, can be obtained by using the cyanine dyes of the present invention as a light absorbent.

### Example 5

### Optical recording medium

As a light absorbent, the either cyanine dye in Table 1 was admixed with TFP to give a concentration of 2.0 w/w %. To the mixture was added, as a light resistance improver, 0.4 w/w % of a known nitroso compound represented by Chemical Formula 24 and ultrasonically dissolved. The resulting solution was homogeneously coated in a conventional manner onto one side of a polycarbonate disc substrate, 12 cm in diameter and 1.2 mm in thickness, to which concaves for expressing synchronizing signals and addresses of tracks and sectors had been transferred to the track's internal circuit and dried to form a recording layer, 300 nm in thickness. Thereafter, the substrate was spattered with silver to form a reflection layer, 100 nm in thickness, to be closely attached to the surface of the recording layer, and then the reflection layer was homogeneously coated with "UNIDIC SD17", as a known ultraviolet ray hardening resin commercialized by Dainippon Ink and Chemicals, Inc., Tokyo, Japan, and irradiated to form a protection layer to be closely attached on the surface of the reflection layer, followed by obtaining an optical recording medium.

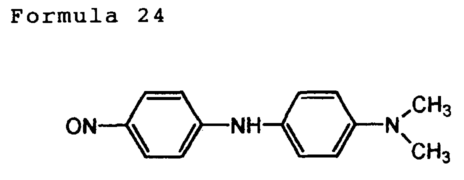

After that, the optical recording medium of this Example can be recorded in a conventional manner 3T and 11T signals (T=4.3218 MHz) in a liner velocity of 9.6 m/sec, eight times as fast as a normal velocity of CD-Rs, through an optical pickup with a wavelength of 780 nm and a wrighting output of 17 mV, respectively. The optical recording media was measured a reflectance, a modulation rate, and 3T jitter and a symmetry in a pit-formed part, a discrepancy between 3T and 11T signals in an average reading output. Table 2 shows the results.

**Table 2**

| Cyanine dye | Reflectance | Modulation rate(%) | | 3T Jitter | Symmetry |
|---|---|---|---|---|---|
| | (%) | 11T | 3T | (nanosecound) | (%) |
| Chemical Formula 6 | 65 | 85 | 49 | 20 | -1 |
| Chemical Formula 9 | 67 | 85 | 47 | 20 | 4 |
| Chemical Formula 14 | 68 | 84 | 45 | 20 | 4 |
| Chemical Formula 15 | 66 | 84 | 44 | 18 | 0 |

As shown in the results in Table 2, the optical recording media of this Example answered a standard which was required for CD-Rs in all measured items. The optical recording media of this Example have a recording capacity of over 600 MB and can be written large amounts of informations of documents, images and voices at a relatively high-density through an optical pickup by laser elements with oscillation wavelengths around 780 nm. When a recording surface of the recording media of this Example, which had been recorded informations, was observed by an electron microscope, the cyanine dye when formed in a crystal was not observed at all and minute pits of which size were about 1 µm were regularly formed at high-density.

As described above, the present invention was made based on the establishment of novel unsymmetric indolenin pentamethine cyanine dyes and their industrially usable features. When formed into a thin layer, the cyanine dyes of the present invention are sensitive to laser beams with wavelengths of 850 nm or shorter. Most of the dyes of the present invention have only decomposition points or those which are undistinguishable from melting points, have relatively-higher decomposition points, have higher heat resistance, and promptly decompose at temperatures around the decomposition points. Accordingly, the cyanine dyes of the present invention can be advantageously useful as a light absorbent in optical recording media in which stable pits should be promptly formed on a limited recording surface, for example optical recording media such as CD-Rs, particularly high-speed-recordable-type CD-Rs.

The cyanine dyes with such usefulness can be obtained in a desired yield through a step of reacting 3,3-dimethyl indolenium compounds having an active methyl group with 2-(1,3-butadienyl)-3,3-dimethyl indolenium compounds having an appropriate leaving group.

The present invention with such outstanding effects and functions is a significant invention that will greatly contribute to this art.

While what are at present considered to be the preferred embodiments of the invention have been described, it will be understood that various modifications may be made therein, and the appended claims are intended to cover all such modifications as fall within the true spirits and scope of the invention.

## Claims

1. An unsymmetrical indolenin pentamethine cyanine dye which is represented by Formula 1 or 2: 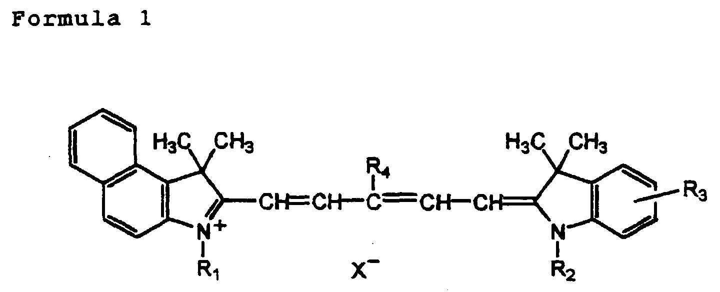 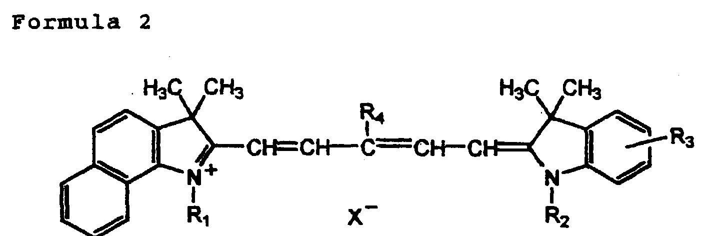 wherein Formulae 1 and 2,
R₁ and R₂ represent an identical or different alkyl group which may have a substituent;
R₃ represents a substituent selected from the group consisting of nitro, cyano, trifluoromethyl, trifluoromethoxy, carbonate, carbonic acid amide, sulfonamide and alkylsulfonyl;
R₄ represents hydrogen or a substituent selected from the group consisting of halogens and methyl; and
X⁻ represents a counter ion.

2. An unsymmetrical indolenin pentamethine cyanine dye according to claim 1, which absorbs visible light with a wavelength of around 780 nm when formed in a thin layer.

3. An unsymmetrical indolenin pentamethine cyanine dye according to claim 1 or claim 2, which has a decomposition point of not lower than 240°C.

4. An unsymmetrical indolenin pentamethine cyanine dye according to any preceding claim, which has only a decomposition point or one that is not easily distinguishable from its melting point.

5. A light absorbent comprising an unsymmetrical indolenin pentamethine cyanine dye as defined in any of claims 1 to 4.

6. A light absorbent according to claim 5, which additionally contains one or more appropriate light-resistance improvers.

7. A light absorbent according to claim 5 or claim 6, which further contains one or more other dye compounds which are sensitive to visible light.

8. A light absorbent according to any of claims 5 to 7, which is sensitive to a laser beam with a wavelength of around 780 nm when formed in a thin layer.

9. An optical recording medium comprising an unsymmetrical indolenin pentamethine cyanine dye as defined in any of claims 1 to 4.

10. An optical recording medium according to claim 9, which additionally contains one or more appropriate light-resistance improvers.

11. An optical recording medium according to claim 9 or claim 10, which further contains one or more other dye compounds which are sensitive to visible light.

12. A process for producing an unsymmetrical indolenin pentamethine cyanine dye, comprising a step of reacting a compound, represented by Formula 3 or 4 having R₁ and R₄ which correspond to Formula 1 or 2, with a compound represented by Formula 5 having R₂ and R₃ which correspond to Formula 1 or 2: 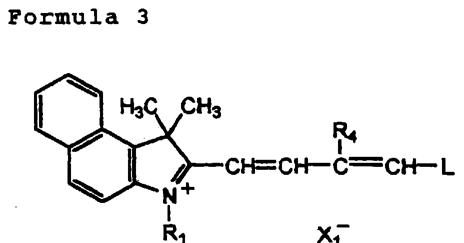 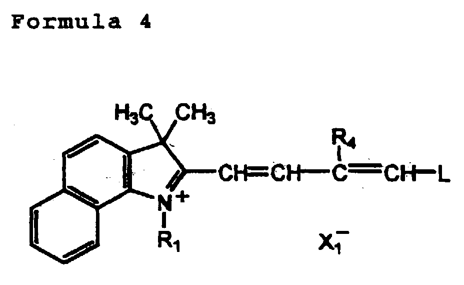 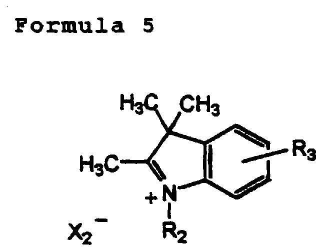 wherein, X₁⁻ and X₂⁻ represent an appropriate counter ion, respectively, and L represents an appropriate leaving group.
